(19) Europäisches Patentamt / European Patent Office / Office européen des brevets

(11) **EP 3 456 305 A1**

(12) **EUROPEAN PATENT APPLICATION**
published in accordance with Art. 153(4) EPC

(43) Date of publication:
**20.03.2019 Bulletin 2019/12**

(51) Int Cl.:
**A61H 1/02** *(2006.01)*　　**A61F 2/72** *(2006.01)*

(21) Application number: **17796261.0**

(22) Date of filing: **15.05.2017**

(86) International application number:
**PCT/JP2017/018216**

(87) International publication number:
**WO 2017/195903 (16.11.2017 Gazette 2017/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.05.2016　JP 2016097345**

(71) Applicant: **Keio University**
**Tokyo 108-8345 (JP)**

(72) Inventors:
- **USHIBA, Junichi**
  **Yokohama-shi**
  **Kanagawa 223-8522 (JP)**
- **LIU, Fumio**
  **Tokyo 160-8582 (JP)**

- **TAKASAKI, Kenichi**
  **Yokohama-shi**
  **Kanagawa 223-8522 (JP)**
- **NISHIMOTO, Atsuko**
  **Tokyo 160-8582 (JP)**
- **HIRAMOTO, Miho**
  **Tokyo 160-8582 (JP)**
- **MIZUNO, Katsuhiro**
  **Tokyo 160-8582 (JP)**
- **LIU, Meigen**
  **Tokyo 160-8582 (JP)**
- **FUJIWARA, Toshiyuki**
  **Tokyo 160-8582 (JP)**

(74) Representative: **Grünecker Patent- und Rechtsanwälte**
**PartG mbB**
**Leopoldstraße 4**
**80802 München (DE)**

(54) **BIOMETRIC INFORMATION PROCESSING DEVICE, BIOMETRIC INFORMATION PROCESSING METHOD AND PROGRAM**

(57)　The objective of the present invention is to make it possible to select and train a brain region appropriate for causing a body part to function. A biometric information processing device 1 is provided with a brain wave detecting unit 10, a control unit 20, and a movement assisting unit 30. The brain wave detecting unit 10 detects biometric information in at least one brain region from among a plurality of brain regions that can be selected in accordance with the body part for which function is to be recovered or improved. On the basis of the biometric information in the brain region detected by the brain wave detecting unit 10, the control unit 20 determines a brain activity state, including the location of a brain region that is activated in a subject who attempts to move the body part, and the level to which the brain region is activated. When the control unit 20 determines that the brain activity state conforms to predetermined conditions, the movement assisting unit 30 executes a predetermined movement.

FIG. 3

**Description**

TECHNICAL FIELD

The present invention relates to a biometric information processing device, a biometric information processing method, and a program.

BACKGROUND ART

According to a conventionally known technique, rehabilitation using a brain-machine interface (BMI) has been conducted on a patient with hemiplegia resulting from a stroke, thereby encouraging the recovery of the patient from paralysis. For example, patent document 1 discloses a technique for rehabilitation with an exoskeleton robot using the BMI.

Patent Document 1: Japanese Unexamined Patent Application, Publication No. 2014-104549

DISCLOSURE OF THE INVENTION

Problems to be Solved by the Invention

According to the conventional technique for rehabilitation using the BMI, as long as recovery from hemiplegia can be achieved by activating a brain nerve pathway connected to a body part with hemiplegia, the purpose of the rehabilitation is regarded as being attained. In some cases, however, multiple brain nerve pathways remain connected to the body part with hemiplegia. In such cases, determining a brain nerve pathway to be activated and recovering the body part from the hemiplegia is important in encouraging the recovery of the entire body of a patient. If the patient suffers from hemiplegia at a left finger and a left shoulder and a brain nerve pathway connected to a particular brain region in a right brain is activated to recover the left shoulder from hemiplegia, if the left finger is connected only to the same brain region via a brain nerve pathway, for example, a resource conflict is caused in the brain region. Hence, it becomes difficult to encourage the recovery of the left finger from the paralysis. Specifically, the conventional technique has difficulty in selecting and training an appropriate brain region for making a body part functional.

A purpose of the present invention is to enable the selection and training of an appropriate brain region for making a body part functional.

Means for Solving the Problems

To attain the foregoing purpose, a biometric information processing device according to one aspect of the present invention includes:

a biometric information detecting unit that detects biometric information from at least one of multiple selectable brain regions in accordance with the body part that is to become a target of function recovery or function improvement;
a determination unit that determines an activated state in a brain including the position of the activated brain region of a test subject having tried to make a motion at the body part and the level of the activation in the brain region based on the biometric information from the brain region detected by the biometric information detecting unit; and
an output unit that makes a predetermined motion if the determination unit determines that the activated state in the brain conforms to a predetermined condition.

Effects of the Invention

The present invention enables the selection and training of an appropriate brain region for making a body part functional.

BRIEF DESCRIPTION OF THE DRAWINGS

Fig. 1 is a schematic view showing the principles of rehabilitation using a BMI.
Fig. 2 is a schematic view showing how a finger and a shoulder relate to brain nerve pathways governing the finger and the shoulder.
Fig. 3 is a schematic view showing the entire configuration of a biometric information processing device according to the present invention.

Fig. 4 is a block diagram showing the configuration of an information processor forming a control unit.

Fig. 5 is a schematic view showing the configuration of a movement assisting unit.

Fig. 6 is a schematic view showing a state in which, while a patient tries to elevate a left shoulder, an ipsilateral brain region of the patient is activated.

Fig. 7 is a schematic view showing an example of how a particular brain region is activated by training.

Fig. 8 is a flowchart showing a flow of brain activated state determination processing.

Fig. 9 shows a specific example of shoulder elevation BMI training on a stroke patient with hemiplegia using ERD on an ipsilateral side (undamaged side).

Fig. 10 shows a change in brain waves of one undamaged person between a state before and a state after the shoulder elevation BMI training conducted for three days continuously.

Fig. 11 shows a change in brain waves of one patient with hemiplegia between a state before and a state after the shoulder elevation BMI training conducted for three days continuously.

Fig. 12 shows results of clinical indicators obtained about one patient with hemiplegia in a state before and a state after the shoulder elevation BMI training conducted for three days continuously.

Fig. 13 shows a change in brain waves of one representative test subject among five stroke patients with severe hemiplegia between a state before and a state after the shoulder elevation BMI training conducted for seven days continuously.

Fig. 14 shows a change in a laterality index of the representative test subject in Fig. 13 between a state before and a state after the shoulder elevation BMI training conducted for seven days continuously.

Fig. 15 shows a change in a voluntary electromyogram of the front section of a deltoid of the representative test subject in Fig. 13.

Fig. 16 shows a change in a modulation index (MI) in the electromyogram of the front section of the deltoid.

Fig. 17 shows changes in FMA-U/E (FMA scores of items of assessment in upper limbs) among the five test subjects.

Fig. 18 is a schematic view showing a change in the function of a shoulder corresponding to a paralyzed limb of a representative test subject between a state before and a state after training.

Fig. 19 is a flowchart showing a flow of brain region selection processing.

PREFERRED MODE FOR CARRYING OUT THE INVENTION

An embodiment of the present invention will be described below by referring to the drawings.

[Basic idea of present invention]

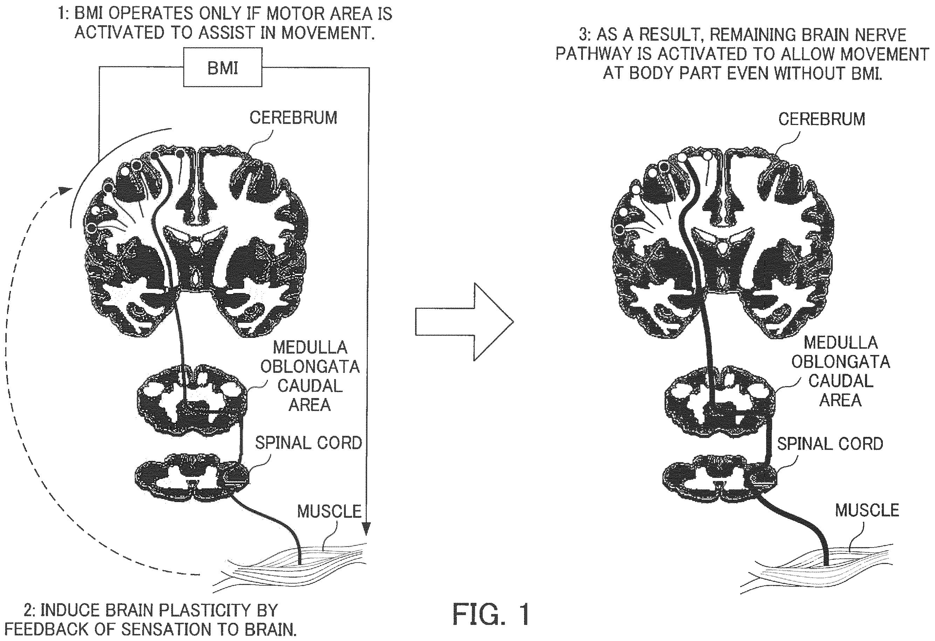

Fig. 1 is a schematic view showing the principles of rehabilitation using a BMI. Fig. 1 shows a cerebrum, a medulla oblongata caudal area, a spinal cord, and a muscle relating to a brain nerve pathway. Brain regions in the cerebrum are shown as circles. The circles showing brain regions include a black circle indicating that a brain region is not activated, and a white circle indicating that a brain region is activated. During the rehabilitation using the BMI, an activated state in a brain region to become a target (motor area, for example) is acquired. Only if this brain region is activated, when a patient tries to move a body part with hemiplegia, the BMI such as a robot or a myoelectric stimulus is actuated to assist in the movement of the body part. Then, a kinesthetic sensation at the assisted body part is fed back to a brain to induce the plasticity of the brain. Such a process is repeated to activate a remaining brain nerve pathway. As a result, the patient becomes capable of making movement at the body part having suffered from hemiplegia even without the BMI.

According to a conventional technique for rehabilitation using a BMI, excitability (activated state) in a somatosensory motor cortex is estimated based on a scalp electroencephalogram opposite (contralateral) to a right or left paralyzed side of a body, and feedback dependent on the level of the estimated excitability is given, thereby achieving recovery of the function of a stroke patient in making movement at a paralyzed hand. A particular frequency component in a brain wave is reduced almost simultaneously with the occurrence of an event such as voluntary movement or a stimulus, which is called event-related desynchronization (ERD). ERD can be used as an index of excitability in a somatosensory motor cortex. According to the foregoing conventional technique for rehabilitation using the BMI, however, the contralateral brain region used in this technique corresponds to a brain on a damaged side of the stroke patient. Hence, resources in this brain region are considered to be restricted.

The rehabilitation is conducted using the contralateral brain region for the reason that a part such as a finger mainly governed by a contralateral brain nerve pathway is assumed as a target of the conventional rehabilitation using a BMI. By contrast, what is important for restoring motions for the daily life of a stroke patient with hemiplegia is not only recovering motion at a finger (such as gripping motion) but also recovering the motion at a shoulder and an elbow (such as reaching motion of bending and extending the shoulder and the elbow). Unlike motion at a finger, motion at a shoulder and an elbow is governed not only by a contralateral brain nerve pathway but also by an ipsilateral brain nerve pathway making up a fixed ratio.

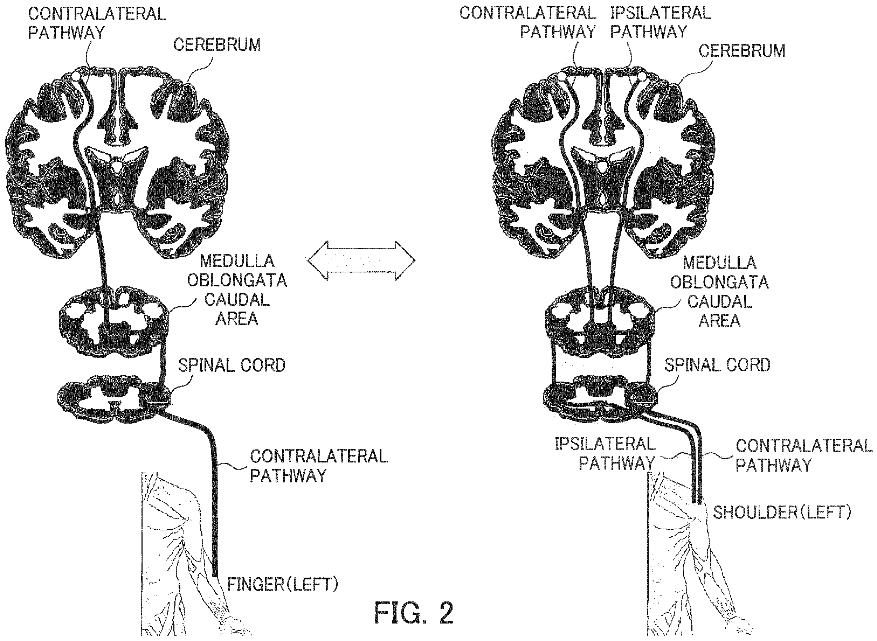

Fig. 2 is a schematic view showing how a finger and a shoulder relate to a brain nerve pathway governing the finger and the shoulder. As shown in Fig. 2, the finger is substantially governed by a contralateral brain nerve pathway, whereas the shoulder is governed by the contralateral brain nerve pathway and an ipsilateral brain nerve system making up ratios nearly equal to each other. In this regard, according to a BMI technique of the present invention for switching a brain information channel (a brain nerve pathway used for function recovery), excitability in a somatosensory motor cortex is estimated based on a scalp electroencephalogram on an ipsilateral side corresponding to an undamaged side of a patient with hemiplegia, and feedback dependent on the level of the estimated excitability is given such as assistance in the movement of elevating a shoulder corresponding to a paralyzed upper limb using an upper limb exoskeleton robot or an electric stimulus on an upper limb proximal muscle using an electric stimulator.

In this way, the present invention encourages an increase in excitability in an ipsilateral somatosensory motor cortex and resultant recovery of the function of a paralyzed upper limb centered on a proximal muscle. Specifically, a brain nerve pathway to be used for function recovery of a body part is switched to a brain information channel extending from the ipsilateral somatosensory motor cortex to the paralyzed upper limb. This enables the selection and training of an appropriate brain region for making a body part functional.

[Configuration]

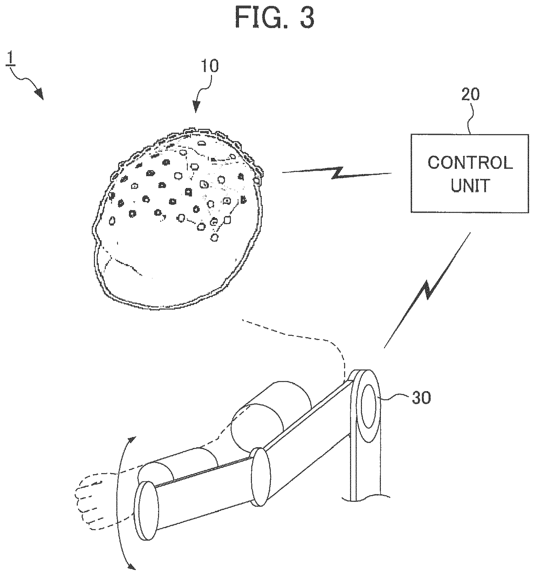

Fig. 3 is a schematic view showing the entire configuration of a biometric information processing device 1 according to the present invention. The biometric information processing device 1 includes a brain wave detecting unit 10 (biometric information detecting unit), a control unit 20 (determination unit), and a movement assisting unit 30 (output unit). These units are configured to be capable of communicating with each other through wired communication or wireless communication.

The brain wave detecting unit 10 includes an electrode for detecting a brain wave as biometric information on a brain based on potential change at a scalp. In this embodiment, the brain wave detecting unit 10 is configured as multiple electrodes arranged in a matrix on the entire scalp. Based on a result of detection by the brain wave detecting unit 10, the control unit 20 can acquire an activated state in a brain including the position of an activated brain region and the level of the activation. A signal of the brain wave detected by the brain wave detecting unit 10 is amplified to a signal level appropriate for processing at a subsequent stage and then transmitted to the control unit 20. The activated state in the brain region may be acquired not only through detection of a brain wave as biometric information about the brain but also through detection of a blood flow rate in the brain using a sensor for detecting a change in a brain blood flow by means of near-infrared spectrophotometry.

The control unit 20 is configured using an information processor such as a personal computer (PC), a tablet terminal, or a smartphone. The control unit 20 performs brain activated state determination processing described later to control the movement assisting unit 30 based on a result of the detection by the brain wave detecting unit 10.

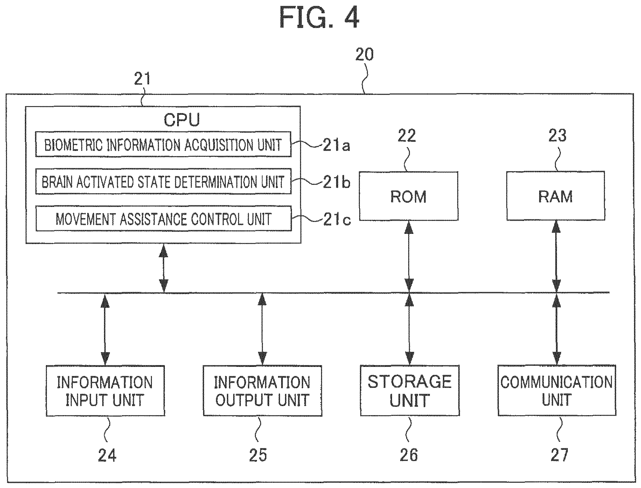

Fig. 4 is a block diagram showing the configuration of the information processor forming the control unit 20. As shown in Fig. 4, the control unit 20 includes a central processing unit (CPU) 21, a read-only memory (ROM) 22, a random access memory (RAM) 23, an information input unit 24, an information output unit 25, a storage unit 26, and a communication unit 27.

The CPU 21 performs various types of processing by following a program stored in the ROM 22 or the storage unit 26. The ROM 22 stores various types of programs for controlling the biometric information processing device 1. The RAM 23 stores data, etc. used for implementation of the various types of processing by the CPU 21.

The information input unit 24 is configured using an input device such as a keyboard and a mouse or a touch panel. The information input unit 24 is used for inputting various types of information depending on the user's instructive operation. The information output unit 25 is configured using a display or a speaker, and used for displaying information or outputting voice under control by the CPU 21. The storage unit 26 is configured using a storage unit such as a hard disk and stores various types of data or various programs to be used by the biometric information processing device 1. The communication unit 27 is used for communication with a different device through wired communication using a universal serial bus (USB) cable or wireless communication such as Bluetooth (registered trademark).

In the biometric information processing device 1 having the foregoing configuration, the CPU 21 executes a program for the brain activated state determination processing. By doing so, functional configurations including a biometric information acquisition unit 21a, a brain activated state determination unit 21b, and a movement assistance control unit 21c are formed in the CPU 21.

The biometric information acquisition unit 21a acquires data on a brain wave from a brain region detected by the brain wave detecting unit 10 as biometric information indicating an activated state in a brain. In an example described in this embodiment, a patient with hemiplegia at a left finger and a left shoulder is a target of rehabilitation for recovering the function of the left shoulder. The biometric information acquisition unit 21a is to acquire a brain wave from a brain region in a somatosensory motor cortex in a right brain and a brain wave from a brain region in a somatosensory motor cortex in a left brain. In this case, the brain activated state determination processing can be performed if at least the brain wave from the somatosensory motor cortex in the right brain and the brain wave from the somatosensory motor cortex in the left brain are acquired. Alternatively, brain waves from brain regions detected from all of the electrodes of the brain wave detecting unit 10 may be acquired, and the absence of unnecessary activation occurring in brain regions other than a brain region that is a target of rehabilitation may also be determined.

The brain activated state determination unit 21b determines whether the activated state in the brain conforms to a predetermined condition for running the movement assisting unit 30 (hereinafter called a "movement assistance condition"), based on the data on the brain wave from the brain region acquired by the biometric information acquisition unit 21a. In this embodiment, the function of the left shoulder is a target of rehabilitation for the patient with hemiplegia at the left finger and the left shoulder. Thus, the brain region in the somatosensory motor cortex in the right brain is used for recovery of the function of the left finger, while the brain region in the somatosensory motor cortex in the ipsilateral left brain is used for recovery of the function of the left shoulder. For this reason, the movement assistance condition is set to be fulfilled if a particular frequency component in a detected brain wave is reduced at a position on a scalp corresponding to the brain region in the somatosensory motor cortex in the left brain (specifically, if ERD occurs). More specifically, the movement assistance condition is set to be fulfilled if the amplitude of vibration in a brain wave containing a frequency from 8 to 13 Hz as a major component (mu rhythm) becomes smaller than a set threshold. The threshold for the amplitude of vibration from 8 to 13 Hz in a brain wave (mu rhythm) is settable at a value dependent on the ERD of an individual patient.

If the brain activated state determination unit 21b determines that the data on the brain wave from the brain region acquired by the biometric information acquisition unit 21a conforms to the movement assistance condition, the movement assistance control unit 21c outputs an instruction signal for driving the movement assisting unit 30. The movement assisting unit 30 supports an arm with hemiplegia of the patient. In response to input of the instruction signal instructing driving of the movement assisting unit 30 from the movement assistance control unit 21c, the movement assisting unit 30 assists the patient in the motion of elevating the arm with the hemiplegia using an actuator.

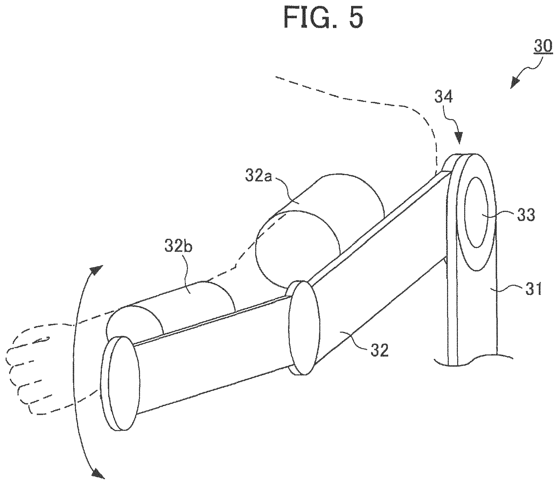

Fig. 5 is a schematic view showing the configuration of the movement assisting unit 30. As shown in Fig. 5, the movement assisting unit 30 is configured as an upper limb exoskeleton robot including a body support part 31, an arm part 32, a coupling part 33, and an actuator 34. The body support part 31 is fixed to a chair for seating a patient and holds the arm part 32 rotatably via the coupling part 33. The actuator 34 causes the body support part 31 and the arm part 32 to rotate relative to each other.

The arm part 32 is an arm member arranged to extend along an arm of a patient. The arm part 32 has a section corresponding to an upper arm and a section corresponding to a forearm of the patient. These sections are continuous with each other and bent at a predetermined angle (corresponding to an angle to which the patient bends his or her elbow slightly). The upper end of the section of the arm part 32 corresponding to the upper arm is coupled by the coupling part 33 so as to be vertically rotatable relative to the body support part 31. The section corresponding to the upper arm and the section corresponding to the forearm of the arm part 32 are provided with a belt member 32a and a belt member 32b respectively for supporting the upper arm and the forearm of the patient. The coupling part 33 is for coupling the arm part 32 so as to allow the arm part 32 to vertically rotate relative to the body support part 31. The actuator 34 outputs driving power for causing the arm part 32 to rotate relative to the body support part 31.

The biometric information processing device 1 having the foregoing configuration detects a brain wave from a patient trying to elevate a left shoulder and determines whether a brain region (here, an ipsilateral brain region) that is a target of rehabilitation is activated.

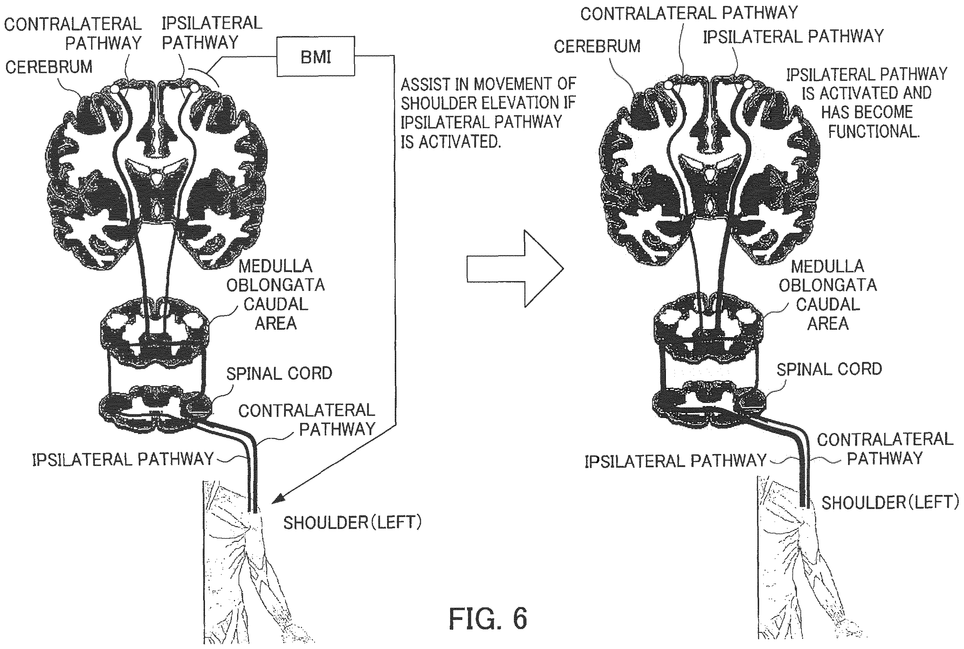

Fig. 6 is a schematic view showing a state in which, while a patient tries to elevate a left shoulder, an ipsilateral brain region of the patient is activated. If the patient tries to make a particular motion such as elevation of the left shoulder, any brain region connected to a body part responsible for the motion via a brain nerve pathway is activated. In this embodiment, as described above, a brain nerve pathway to be used for the particular motion (here, elevation of the left shoulder) is selected. When the patient tries to make the particular motion, the biometric information processing device 1 trains the patient so as to activate a brain region connected to the selected brain nerve pathway (ipsilateral brain nerve pathway).

Specifically, if a particular brain region is determined to be activated when the patient tries to make the particular motion at a body part with hemiplegia, the biometric information processing device 1 assists in movement using the movement assisting unit 30. Then, a kinesthetic sensation at the assisted body part is fed back to a brain to induce the plasticity of the brain. Such process is repeated to train the patient so as to make a gradual transition from an initial state in which a brain region activated by the attempt to make the particular motion is unfixed to a state in which a targeted brain region is activated. By doing so, the patient even without the assistance from the movement assisting unit 30 becomes capable of making movement at the body part having suffered from hemiplegia using a brain nerve pathway of the patient himself or herself.

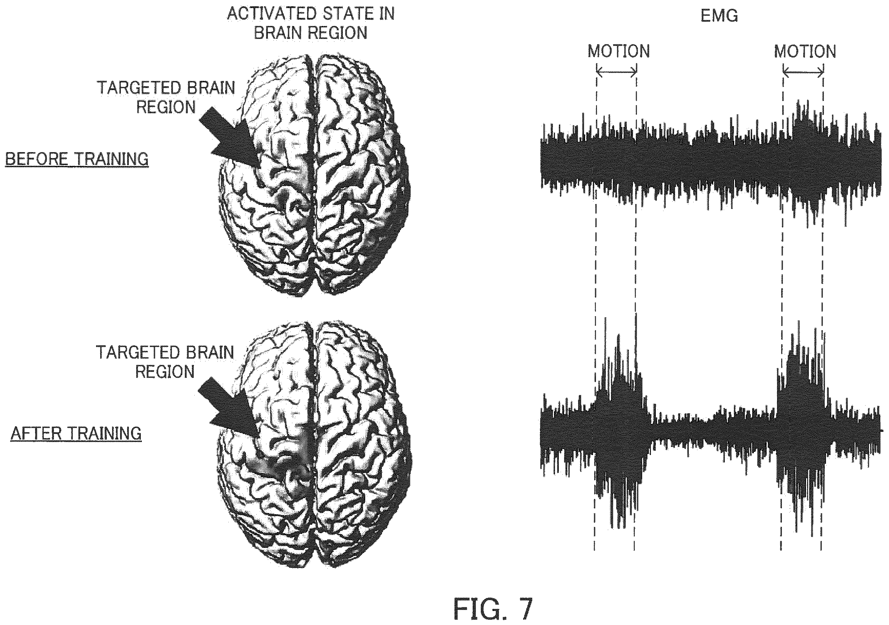

Fig. 7 is a schematic view showing an example of how a particular brain region is activated by training. Fig. 7 includes a schematic view of a brain showing an activated state in a brain region, and an electromyogram (EMG) of a body part (paralyzed muscle) controlled by this brain region. In the schematic view of the brain in Fig. 7, a denser area indicates that a brain region is in a more active state (presence of ERD). As understood from this schematic view, the function of the paralyzed muscle is recovered by activating a brain region in association with the particular motion by training.

[Operation]

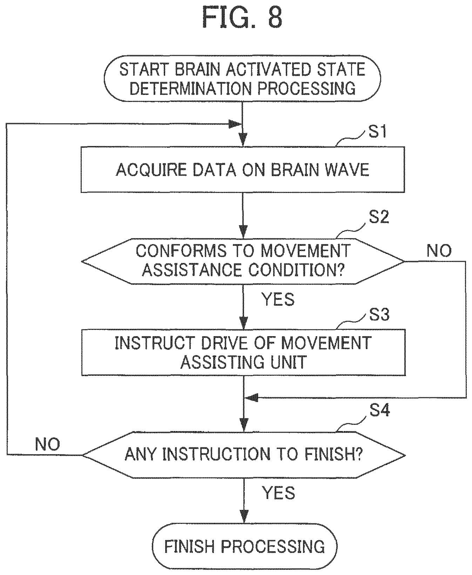

The operation of the biometric information processing device 1 will be described next. Fig. 8 is a flowchart showing a flow of the brain activated state determination processing. The brain activated state determination processing is started by input of an instruction to perform the brain activated state determination processing through the information input unit 24.

In step S1, the biometric information acquisition unit 21a acquires data on a brain wave from a brain region detected by the brain wave detecting unit 10 as biometric information indicating an activated state in a brain. In step S2, the brain activated state determination unit 21b determines whether the activated state in the brain conforms to a predetermined condition for running the movement assisting unit 30 (movement assistance condition), based on the data on the brain wave from the brain region acquired by the biometric information acquisition unit 21a. If the activated state in the brain does not conform to the movement assistance condition, a determination NO is made in step S2, and the processing goes to step S4. If the activated state in the brain conforms to the movement assistance condition, a determination YES is made in step S2, and the processing goes to step S3.

In step S3, the movement assistance control unit 21c outputs an instruction signal for driving the movement assisting unit 30. In response to this, the actuator 34 of the movement assisting unit 30 outputs driving power for causing the arm part 32 to rotate relative to the body support part 31. Specifically, the arm part 32 of the movement assisting unit 30 is rotated upward relative to the body support part 31 to assist a patient in the movement of elevating a left shoulder.

In step S4, the brain activated state determination unit 21b determines whether an instruction to finish the brain activated state determination processing has been input. In the absence of input of an instruction to finish the brain activated state determination processing, a determination NO is made in step S4, and the processing goes to step S1. In the presence of input of an instruction to finish the brain activated state determination processing, a determination YES is made in step S4. Then, the brain activated state determination processing is finished.

[Advantageous effect of embodiment]

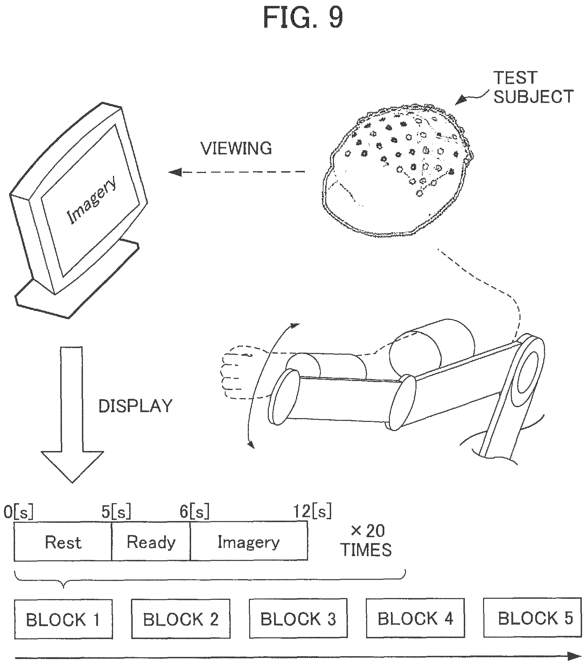

Fig. 9 shows a specific example of shoulder elevation BMI training on a stroke patient with hemiplegia using ERD on an ipsilateral side (undamaged side). In the example shown in Fig. 9, by employing an electrode arrangement based on the international 10-20 method, the ERD of a brain wave detected by an electrode at a C3 (left brain) position and that of a brain wave detected by an electrode at a C4 (right brain) position were used as biomarkers for shoulder elevation movement resulting from brain activities on the corresponding ipsilateral sides. Each of the test subjects (a patient and an undamaged person) tried to make the motion of elevating a shoulder corresponding to a paralyzed side. If the ipsilateral ERD exceeded a fixed threshold, the shoulder was passively elevated by an upper limb exoskeleton robot attached to each test subject.

In this example, a patient with hemiplegia performed the process 20 times repeatedly multiplied by five blocks in a single day, thereby performing the process 100 times in total. By doing so, the patient with hemiplegia was trained to make movement at a paralyzed upper limb. The shoulder elevation BMI training was conducted in a single day on three undamaged persons and four patients with hemiplegia. Further, the shoulder elevation BMI training was conducted for three continuous days on one undamaged person and one patient with hemiplegia, thereby acquiring experimental results. The high dense electroencephalograph (128 ch, available from Electrical Geodesies, Inc.) was used as a brain wave measurement device.

With the exoskeleton robot attached to the test subject, the test subject viewed a screen on which the characters Rest (five seconds), Read (one second), and Imagery (six seconds) appeared in that order. When Imagery appeared, the test subject imagined an "image of bending a shoulder 90 degrees by extending an elbow."

When the ERD of an ipsilateral (undamaged) somatosensory motor cortex during the imagining of movement became 30% or more, an upper limb of the test subject was passively bent by the upper limb exoskeleton robot. This process was performed 20 times multiplied by five blocks per session. As a result, in this example, the imagining of shoulder elevation movement accompanied by ipsilateral ERD exceeding the fixed threshold during the shoulder elevation BMI training in a single day achieved success rates of 56% ±4% for the undamaged persons, and success rates of 79% ± 21% for the patients. This shows that the shoulder elevation BMI training using the ipsilateral ERD was feasible in all cases.

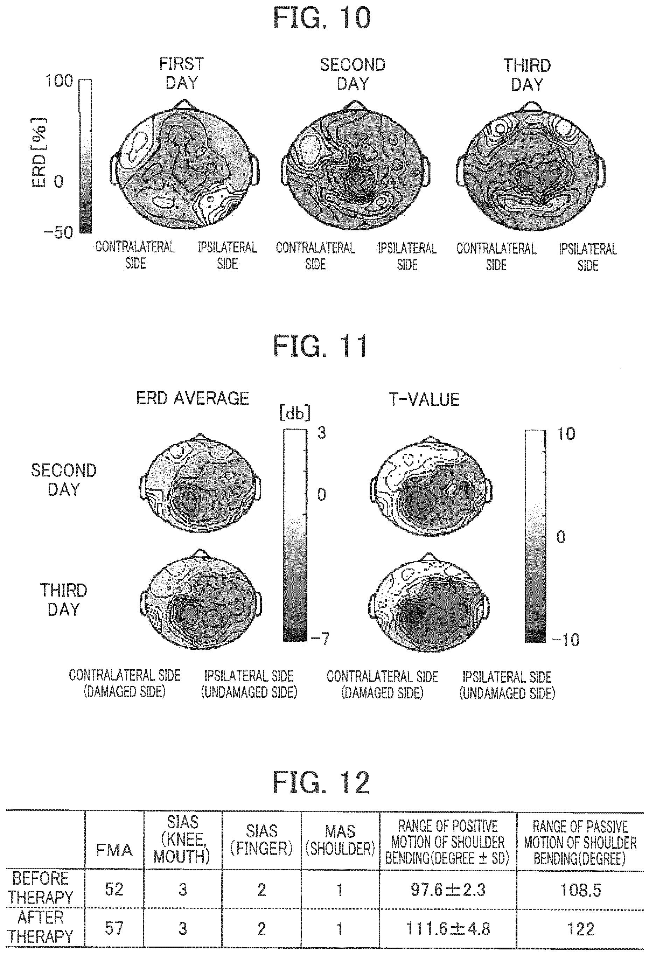

Fig. 10 shows a change in brain waves of one undamaged person between a state before and a state after the shoulder elevation BMI training conducted for three days continuously. Fig. 10 shows ERD averages of corresponding channels of all recorded brain waves in the form of varying densities. A higher density shows more apparent ERD and increased excitability (activated state) in brain activity. A comparison between the first day and the third day shows that ipsilateral ERD during the imagining of shoulder elevation tends to be increased by the shoulder elevation BMI training.

Fig. 11 shows a change in brain waves of one patient with hemiplegia between a state before and a state after the shoulder elevation BMI training conducted for three days continuously. On the first day of the training, a significant increase in muscle tone was recognized at a fiber at the front section of a paralyzed deltoid during the imagining of movement and influence by muscle activity was mixed as noise into a brain wave. Thus, results from the first day were excluded from an analysis result. For this reason, Fig. 11 shows brain waves (activated state in a brain) on the second day and on the third day when the rest state of the fiber at the front section of the paralyzed deltoid was assured during the imagining of movement. The schematic views of the brain on the left side of Fig. 11 show ERD averages. The schematic views of the brain on the right side of Fig. 11 show the T-values of corresponding channels (electrodes) obtained by conducting a T-test using an ERD value in a rest state in comparison to an ERD value during the imagining of movement. As seen from the schematic views of the brain on the right side of Fig. 11, ERD values obtained by each implementation of the test are distributed less as density becomes higher. This shows the occurrence of the ERD more reliably. In this way, the shoulder elevation BMI training conducted on the patient with hemiplegia was recognized to develop a tendency to increase an ipsilateral ERD average and the T-value of the ipsilateral ERD.

Fig. 12 shows results of clinical indicators obtained about one patient with hemiplegia in a state before and a state after the shoulder elevation BMI training conducted for three days continuously. Fugl-Meyer Assessment (FMA) and Stroke Impairment Assessment Set (SIAS) are clinical assessments for a paralyzed upper limb function. Further, Modified Ashworth Scale (MAS) is a clinical measure for evaluating a degree of muscle tone at a paralyzed upper limb. Regarding the FMA, a minimal clinically important difference (MCID) is known to range from 4.25 to 7.25 points. As shown in Fig. 12, improvement in the FMA was recognized in the paralysis at the upper limb of the patient with hemiplegia as a result of the shoulder elevation BMI training conducted for three days. Improvements were also recognized in angles of the shoulder elevation during positive movement and passive movement.

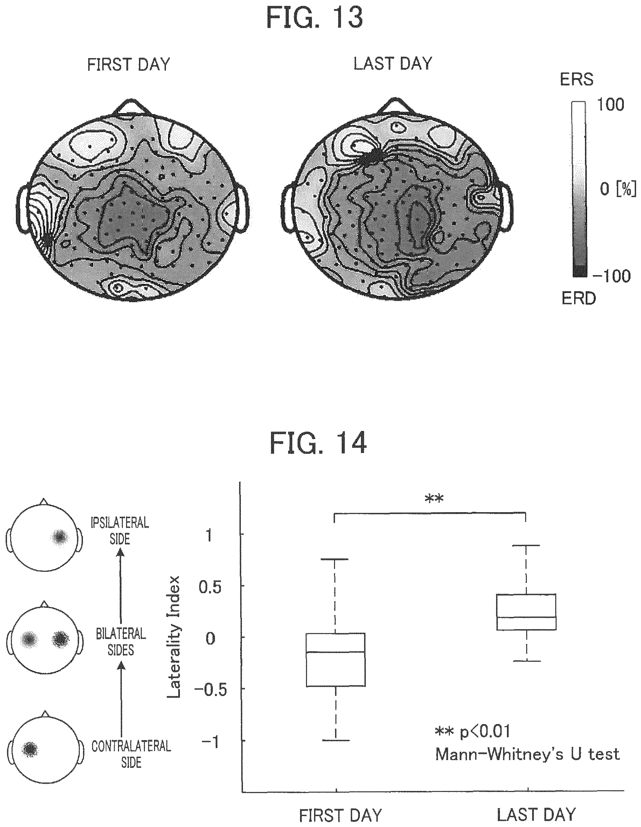

Figs. 13 to 18 show results of BMI training conducted for one hour a day and seven days in total on five stroke patients with severe hemiplegia. A BMI rehabilitation system that is used has the same configuration as that shown in Fig. 9. More specifically, Fig. 13 shows a change in brain waves of one representative test subject among the five stroke patients with severe hemiplegia between a state before and a state after the shoulder elevation BMI training conducted for seven days continuously. Fig. 13 shows that ERD becomes more apparent in an area with higher density, whereas event-related synchronization (ERS) becomes more apparent in an area with lower density. Fig. 14 shows a change in a laterality index of the representative test subject in Fig. 13 between a state before and a state after the shoulder elevation BMI training conducted for seven days continuously. The laterality index in Fig. 14 is defined as follows:

$$\text{laterality index} = (\text{contralateral ERD} - \text{ipsilateral ERD}) / (|\text{contralateral ERD}| + |\text{ipsilateral ERD}|).$$

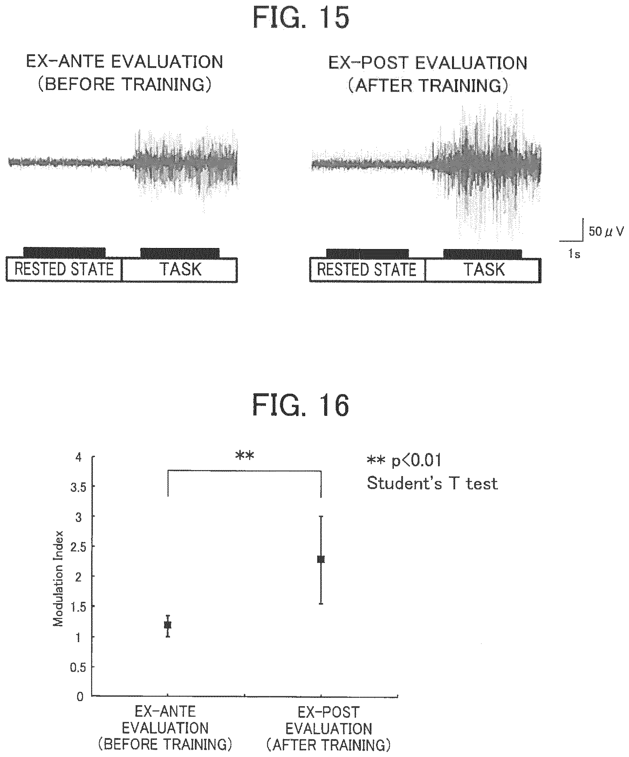

The BMI training shown in Figs. 13 to 18 was intended to detect a brain wave by affixing an electrode at a position, among a motor area in a left hemisphere and a motor area in a right hemisphere connected to a muscle at a paralyzed right shoulder, near a motor area in a right hemisphere, and to train the function of a nerve pathway (a pathway ipsilateral to a paralyzed limb) extending downward from the motor area in the right hemisphere to the muscle at the shoulder. As a result, a movement-related brain wave (an area of a high density in Fig. 13) generated when the shoulder elevation movement was intended was replaced by a brain wave on the right side used in the training using the BMI. Based on this result, the BMI according to this embodiment intended to activate a function through pathway selection is considered to have achieved the objective of selectively activating a nerve pathway extending downward from the motor area in the right hemisphere to the muscle at the shoulder. Comparable significant changes in brain waves were also confirmed in two out of the five test subjects. Fig. 15 shows a change in a voluntary electromyogram about the front section of a deltoid of the representative test subject in Fig. 13. Fig. 16 shows a change in a modulation index (MI) in the electromyogram of the front section of the deltoid. The modulation index MI(t) in Fig. 16 is defined as follows:

$$MI(t) = (1/T_{task}) \sum EMG(t)_{task} / (1/T_{rest}) \sum EMG(t)_{rest}.$$

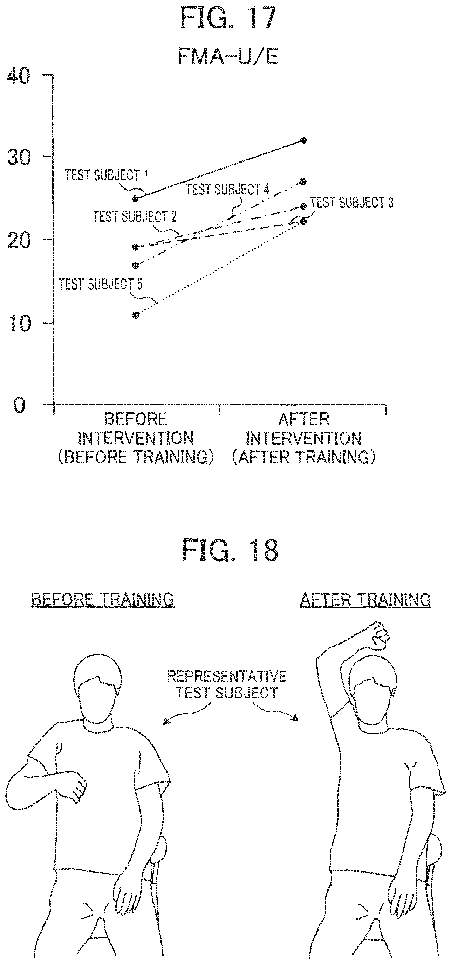

Here, $T_{task}$ is the number of sample data pieces during the imagining of movement, $T_{rest}$ is the number of sample data pieces in a rest state, $EMG(t)_{task}$ is a waveform in an electromyogram during the imagining of movement (during a task), and EMG(t)$_{rest}$ is a waveform in an electromyogram in a rest state. As shown in Figs. 15 and 16, observation of the electromyograms of the front section of the deltoid that becomes active during elevation of the shoulder corresponding to the paralyzed limb shows that the amount of activation increases significantly after the training. This means that, as a result of the fulfillment of the objective of selectively activating a nerve pathway extending downward from the motor area in the right hemisphere to the muscle at the shoulder by the BMI according to this embodiment, voluntary control over a muscle can be exerted more strongly. Comparable significant changes in electromyograms were also confirmed in four out of the five test subjects. Fig. 17 shows changes in FMA-U/E (FMA scores of items of assessment in upper limbs) among the five test subjects. As shown in Fig. 17, clinical therapeutic effects achieved as a result of the foregoing BMI training were evaluated using FMA-U/E as a clinical movement evaluation index, and significant function recoveries were recognized in all cases. Fig. 18 is a schematic view showing a change in the function of a shoulder corresponding to a paralyzed limb of a representative test subject between a state before and a state after training. As shown in Fig. 18, as a result of the implementation of the foregoing BMI training, the paralyzed shoulder is brought to a state of being capable of elevating to an apparently higher position than the position before the training.

As described above, unique effects resulting from the present invention include increased excitability in an ipsilateral somatosensory motor cortex during the imagining of shoulder elevation movement recognized in an undamaged person and a patient with hemiplegia, and resultant effect of recovering the function of a paralyzed upper limb recognized in the patient with hemiplegia. This phenomenon is considered to be brought about by making a switch to a brain information channel (brain nerve pathway) extending from an ipsilateral somatosensory motor cortex to the paralyzed upper limb.

[First modification]

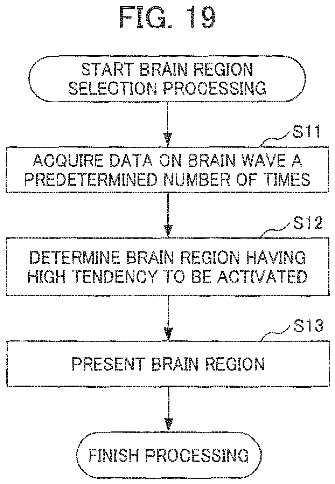

In the foregoing embodiment, before implementation of the brain activated state determination processing, processing (brain region selection processing) can be performed by which a tendency in an activated state in a brain region in the same patient is detected, and based on a result of the detection, a brain region to become a target of rehabilitation is selected. Fig. 19 is a flowchart showing a flow of the brain region selection processing. The brain region selection processing is started by input of an instruction to perform the brain region selection processing through the information input unit 24. In step S11, the biometric information acquisition unit 21a repeats the process of acquiring a brain wave from each brain region of a patient who makes a particular motion at a body part with hemiplegia a predetermined number of times. For example, in step S11, the patient repeats the particular motion five times at the body part with hemiplegia, and the biometric information acquisition unit 21a acquires brain waves from each brain region corresponding to the motion made five times. In step S12, the brain activated state determination unit 21b determines a brain region having a high tendency to be activated by the particular motion at the body part with hemiplegia. In step S13, the brain activated state determination unit 21b presents the brain region (presentation on a display, for example), determined to have a high tendency to be activated by the particular motion at the body part with hemiplegia, as a brain region suitable as a target of rehabilitation. In this step, a suitable brain region to be presented as a target may be a brain region among previously designated brain regions (brain regions limited to an ipsilateral side or selected brain regions not highly activated at the present time but determined to have a high tendency to be activated by training, for example). By performing the brain region selection processing before implementation of the brain activated state determination processing and training, a brain region of the same patient to be activated easily by the particular motion in the training is selected. By doing so, the body function of the patient can be recovered more efficiently.

[Second modification]

In the brain activated state determination processing described in the foregoing embodiment, if an activated state in a brain conforms to the movement assistance condition, the movement assisting unit 30 assists a patient in the movement of elevating a left shoulder. Instead of making the movement assisting unit 30 assist in the movement in response to conformity of the activated state in the brain with the movement assistance condition, an indication may be displayed on a display showing that the activated state in the brain is appropriate. This allows training to be conducted simply without the use of the movement assisting unit 30. Specifically, the patient is allowed to rehabilitate himself or herself at home, for example. In addition to making the movement assisting unit 30 assist in the movement, an indication may be displayed on the display showing that the activated state in the brain is appropriate.

[Third modification]

In the foregoing embodiment, if an activated state in a brain does not conform to the movement assistance condition, a patient may receive a sensory stimulus (such as pain, vibration, pressurization, electric stimulus, or voice) notifying the inappropriateness of the activated state in the brain. Giving such a stimulus makes it possible to retain the training effect in the patient at a higher rate.

The present invention is not limited to the foregoing embodiment, and modifications, improvements, etc. can be added to the present invention appropriately within a range in which the effects of the present invention are achieved. For example, in the foregoing embodiment, in addition to a brain region ipsilateral to a body part with hemiplegia, a brain region may be selected from multiple selectable contralateral brain regions and trained. Alternatively, a brain region contralateral to a body part with hemiplegia and a brain region ipsilateral brain region may be selected, and the selected brain regions may be determined to be brain regions that are targets of rehabilitation. In this way, multiple body parts may be trained. In the case of adults, these selected brain regions can be movement-related regions such as a somatosensory motor cortex, a supplementary motor area, a premotor cortex, and a presupplementary motor area, for example. In the case of children or infants, the selected brain regions can include brain regions such as a sensory area other than movement-related regions, in addition to the movement-related regions. A brain region to be selected from these brain regions is determined appropriately in consideration of the situation of each patient such as the severity of a stroke, the size of a damaged tissue or a damaged site, whether the stroke has occurred during childhood, the presence of brachial plexus palsy (avulsion syndrome), the presence of spinal cord injury, the presence of cerebral palsy, or the presence of neuromuscular intractable disease, for example.

In the example described in the foregoing embodiment, function recovery of a body part of a patient with hemiplegia is intended. However, this is not the only case. More specifically, an undamaged person may be trained for function improvement of a body part by using the biometric information processing device 1.

In the foregoing embodiment, the brain wave detecting unit 10 is configured as electrodes arranged in a matrix over a scalp entirely. However, this is not the only case. More specifically, the brain wave detecting unit 10 may be configured as one electrode or multiple electrodes provided at a position on the scalp corresponding to a brain region selected as a target of rehabilitation. In the example described in the foregoing embodiment, the movement assisting unit 30 is configured as an upper limb exoskeleton robot. However, this is not the only case. More specifically, as long as the movement assisting unit 30 can assist in the movement of a patient, a device of a different configuration is applicable. For example, the movement assisting unit 30 can be configured as an electric stimulator to apply a myoelectric stimulus to a body part with hemiplegia.

The present invention can also be carried out by combining the foregoing embodiment and each of the foregoing modifications. The processing described in the foregoing embodiment can be performed either by hardware or by software. More specifically, as long as the biometric information processing device 1 has a function capable of performing the foregoing processing, a functional configuration and a hardware configuration for fulfilling this function are not limited to those described in the foregoing example. To perform the foregoing processing by software, a program constituting the software is installed on a computer from a network or a storage medium.

The storage medium storing the program is configured using a removable medium to be distributed and is separate from a device body, or is configured using a storage medium incorporated in advance in the device body, for example. The removable medium is configured using a magnetic disk, an optical disk, or a magneto-optical disk, for example. The optical disk is configured using a compact disk read-only memory (CD-ROM), a digital versatile disk (DVD), or a Blu-ray Disk (registered trademark), for example. The magneto-optical disk is configured using a mini-disk (MD), for example. The storage medium incorporated in advance in the device body is configured using a ROM or a hard disk storing a program, for example.

The biometric information processing device 1 having the foregoing configuration includes the brain wave detecting unit 10, the control unit 20, and the movement assisting unit 30. The brain wave detecting unit 10 detects biometric information from at least one of multiple selectable brain regions in accordance with the body part that is to become a target of function recovery or function improvement. The control unit 20 determines an activated state in a brain including the position of an activated brain region of a test subject having tried to make a motion at the body part and the level of the activation in the brain region based on the biometric information from the brain region detected by the brain wave detecting unit 10. The movement assisting unit 30 makes a predetermined motion if the control unit 20 determines that the activated state in the brain conforms to a predetermined condition. As a result, an appropriate brain region for making the body part functional can be selected from multiple selectable brain regions and trained in accordance with the body part that is to become a target of function recovery or function improvement.

The brain wave detecting unit 10 detects biometric information from the brain region ipsilateral to the body part that is to become a target of function recovery or function improvement. As a result, training for making the body part functional can be conducted using a brain region selected from the brain regions ipsilateral to the body part.

The brain wave detecting unit 10 detects biometric information from the brain region contralateral to the body part that is to become a target of function recovery or function improvement. As a result, training for making the body part functional can be conducted using a brain region selected from the brain regions contralateral to the body part.

The brain wave detecting unit 10 detects biometric information from the brain region ipsilateral to a first body part that is to become a target of function recovery or function improvement and detects biometric information from the brain region contralateral to a second body part that is to become a target of function recovery or function improvement.

As a result, training for making multiple body parts functional can be conducted using the brain region selected from the brain regions contralateral to the body part and the brain region selected from the brain regions ipsilateral to the body part.

The brain wave detecting unit 10 detects biometric information from the brain region in a movement-related region in the brain of an adult. As a result, an appropriate selectable brain region can be selected from the brain of an adult for training to make the body part functional.

The brain wave detecting unit 10 detects biometric information from the brain region in a movement-related region and in a region other than a movement-related region including a sensory area in the brain of a child or an infant. As a result, an extensive brain region likely to be activated by training can be selected from the brain of a child or an infant for training to make the body part functional.

The brain wave detecting unit 10 detects biometric information from multiple brain regions of the test subject having tried to make a motion at the body part. The control unit 20 determines the brain region, selected as a target to be associated with the body part from the multiple activated brain regions, to be a determination target to be determined in terms of the activated state in the brain based on a brain wave detected by the brain wave detecting unit 10. As a result, an appropriate brain region of the test subject to be activated by particular motion in the training is selected so that the training for making the body part functional can be conducted more efficiently.

The output unit includes an assisting device (movement assisting unit 30) for assisting in motion at the body part. As a result, a kinesthetic sensation at the body part is fed back to the brain to allow induction of the plasticity of the brain.

The output unit includes a display device (the display of the information output unit 25) for showing whether the activated state in the brain conforms to a predetermined condition. As a result, training for making the body part functional can be conducted simply.

The output unit includes a sensory stimulator that applies a sensory stimulus to the test subject if the activated state in the brain does not conform to the predetermined condition. This makes it possible to retain the training effect in the test subject at a higher rate.

EXPLANATION OF REFERENCE NUMERALS

1 Biometric information processing device, 10 Brain wave detecting unit, 20 Control unit, 21 CPU, 21a Biometric information acquisition unit, 21b Brain activated state determination unit, 21c Movement assistance control unit, 22 ROM, 23 RAM, 24 Information input unit, 25 Information output unit, 26 Storage unit, 27 Communication unit, 30 Movement assisting unit, 31 Body support part, 32 Arm part, 32a, 32b Belt member, 33 Coupling part, 34 Actuator

**Claims**

1. A biometric information processing device comprising: a biometric information detecting unit that identifies at least one of multiple selectable brain regions existing in hemispheres on bilateral sides of a brain in accordance with a body part that is to become a target of function recovery or function improvement at a finger, a shoulder, and an elbow of a patient with hemiplegia, and detects biometric information from the identified brain region; a determination unit that determines an activated state in the brain including the position of the activated brain region of a test subject having tried to make a motion at the body part and the level of the activation in the brain region based on the biometric information detected from the brain region identified by the biometric information detecting unit; and an output unit that makes a predetermined motion if the determination unit determines that the activated state in the brain conforms to a predetermined condition.

2. The biometric information processing device according to claim 1, wherein the biometric information detecting unit detects biometric information from the brain region ipsilateral to the body part that is to become a target of function recovery or function improvement.

3. The biometric information processing device according to claim 1, wherein the biometric information detecting unit detects biometric information from the brain region contralateral to the body part that is to become a target of function recovery or function improvement.

4. The biometric information processing device according to claim 1, wherein the biometric information detecting unit detects biometric information from the brain region ipsilateral to the body part that is to become a target of function recovery or function improvement and the brain region contralateral to the body part that is to become a target of function recovery or function improvement.

5. The biometric information processing device according to claim 1, wherein the biometric information detecting unit detects biometric information from the brain region ipsilateral to the first body part that is to become a target of function recovery or function improvement, and biometric information from the brain region contralateral to the second body part that is to become a target of function recovery or function improvement.

6. The biometric information processing device according to any one of claims 1 to 5, wherein the biometric information detecting unit detects biometric information from the brain region in a movement-related region in the brain of an adult.

7. The biometric information processing device according to any one of claims 1 to 6, wherein the biometric information detecting unit detects biometric information from the brain region in a movement-related region and in a region other than a movement-related region including a sensory area in the brain of a child or an infant.

8. The biometric information processing device according to any one of claims 1 to 7, wherein the biometric information detecting unit detects biometric information from the multiple brain regions of the test subject having tried to make a motion at the body part, and
the determination unit determines the brain region, selected as a target to be associated with the body part from the multiple activated brain regions, to be a determination target to be determined in terms of the activated state in the brain based on a brain wave detected by the biometric information detecting unit.

9. The biometric information processing device according to any one of claims 1 to 7, wherein the output unit includes an assisting device for assisting in the motion of the body part.

10. The biometric information processing device according to any one of claims 1 to 9, wherein the assisting device includes an assisting device for assisting in the motion of an upper arm section.

11. The biometric information processing device according to any one of claims 1 to 10, wherein the output unit includes a display device for showing whether the activated state in the brain conforms to a predetermined condition.

12. The biometric information processing device according to any one of claims 1 to 11, wherein the output unit includes a sensory stimulator that applies a sensory stimulus to the test subject if the activated state in the brain does not conform to a predetermined condition.

13. A biometric information processing method implemented by a biometric information processing device that processes biometric information about a test subject, the method comprising:

a biometric information detecting step of identifying at least one of multiple selectable brain regions existing in hemispheres on bilateral sides of a brain in accordance with a body part that is to become a target of function recovery or function improvement at a finger, a shoulder, and an elbow of a patient with hemiplegia, and detecting biometric information from the identified brain region;
a determination step of determining an activated state in the brain including the position of the activated brain region of the test subject having tried to make a motion at the body part and the level of the activation in the brain region based on the biometric information detected from the brain region identified in the biometric information detecting step; and
an output step of making a predetermined motion if the activated state in the brain is determined to conform to a predetermined condition in the determination step.

14. A program for causing a computer controlling a biometric information processing device that processes biometric information about a test subject to perform:

a biometric information detecting process of identifying at least one of multiple selectable brain regions existing in hemispheres on bilateral sides of a brain in accordance with a body part that is to become a target of function recovery or function improvement at a finger, a shoulder, and an elbow of a patient with hemiplegia, and detecting biometric information from the identified brain region;
a determination process of determining an activated state in the brain including the position of the activated brain region of the test subject having tried to make a motion at the body part and the level of the activation in the brain region based on the biometric information detected from the brain region identified in the biometric information detecting process; and
an output control process of making a predetermined motion if the activated state in the brain is determined to conform to a predetermined condition in the determination process.

1: BMI OPERATES ONLY IF MOTOR AREA IS ACTIVATED TO ASSIST IN MOVEMENT.

BMI

CEREBRUM

MEDULLA OBLONGATA CAUDAL AREA

SPINAL CORD

MUSCLE

2: INDUCE BRAIN PLASTICITY BY FEEDBACK OF SENSATION TO BRAIN.

3: AS A RESULT, REMAINING BRAIN NERVE PATHWAY IS ACTIVATED TO ALLOW MOVEMENT AT BODY PART EVEN WITHOUT BMI.

CEREBRUM

MEDULLA OBLONGATA CAUDAL AREA

SPINAL CORD

MUSCLE

FIG. 1

EP 3 456 305 A1

FIG. 2

# FIG. 3

# FIG. 4

20

21

## CPU

| BIOMETRIC INFORMATION ACQUISITION UNIT | ~21a |
| BRAIN ACTIVATED STATE DETERMINATION UNIT | ~21b |
| MOVEMENT ASSISTANCE CONTROL UNIT | ~21c |

22

ROM

23

RAM

INFORMATION INPUT UNIT

24

INFORMATION OUTPUT UNIT

25

STORAGE UNIT

26

COMMUNICATION UNIT

27

# FIG. 5

FIG. 6

ACTIVATED STATE IN BRAIN REGION

EMG

BEFORE TRAINING

TARGETED BRAIN REGION

MOTION          MOTION

AFTER TRAINING

TARGETED BRAIN REGION

FIG. 7

FIG. 8

```
        ┌─────────────────────────────────┐
        │  START BRAIN ACTIVATED STATE    │
        │    DETERMINATION PROCESSING     │
        └─────────────────────────────────┘
```

S1

ACQUIRE DATA ON BRAIN WAVE

S2

CONFORMS TO MOVEMENT
ASSISTANCE CONDITION?                     NO

YES

S3

INSTRUCT DRIVE OF MOVEMENT
ASSISTING UNIT

S4

NO   ANY INSTRUCTION TO FINISH?

YES

FINISH PROCESSING

# FIG. 9

TEST SUBJECT

VIEWING

Imagery

DISPLAY

| 0[s] | 5[s] | 6[s] | 12[s] |
|------|------|------|-------|
| Rest | Ready | Imagery | |

×20 TIMES

| BLOCK 1 | BLOCK 2 | BLOCK 3 | BLOCK 4 | BLOCK 5 |

# FIG. 10

# FIG. 11

# FIG. 12

| | FMA | SIAS (KNEE, MOUTH) | SIAS (FINGER) | MAS (SHOULDER) | RANGE OF POSITIVE MOTION OF SHOULDER BENDING(DEGREE ± SD) | RANGE OF PASSIVE MOTION OF SHOULDER BENDING(DEGREE) |
|---|---|---|---|---|---|---|
| BEFORE THERAPY | 52 | 3 | 2 | 1 | 97.6±2.3 | 108.5 |
| AFTER THERAPY | 57 | 3 | 2 | 1 | 111.6±4.8 | 122 |

# FIG. 13

FIRST DAY

LAST DAY

ERS

100

0 [%]

−100

ERD

# FIG. 14

IPSILATERAL SIDE

BILATERAL SIDES

CONTRALATERAL SIDE

Laterality Index

**

** p<0.01
Mann-Whitney's U test

FIRST DAY

LAST DAY

# FIG. 15

EX-ANTE EVALUATION
(BEFORE TRAINING)

EX-POST EVALUATION
(AFTER TRAINING)

| RESTED STATE | TASK |

| RESTED STATE | TASK |

50 μV

1s

# FIG. 16

** p<0.01
Student's T test

# FIG. 17

## FMA-U/E

# FIG. 18

# FIG. 19

START BRAIN REGION
SELECTION PROCESSING

S11

ACQUIRE DATA ON BRAIN WAVE A
PREDETERMINED NUMBER OF TIMES

S12

DETERMINE BRAIN REGION HAVING
HIGH TENDENCY TO BE ACTIVATED

S13

PRESENT BRAIN REGION

FINISH PROCESSING

## INTERNATIONAL SEARCH REPORT

International application No.

PCT/JP2017/018216

### A. CLASSIFICATION OF SUBJECT MATTER

*A61H1/02*(2006.01)i, *A61F2/72*(2006.01)i

According to International Patent Classification (IPC) or to both national classification and IPC

### B. FIELDS SEARCHED

Minimum documentation searched (classification system followed by classification symbols)
A61H1/02, B25J1/00-21/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2012-217721 A (Keio University), 12 November 2012 (12.11.2012), | 1,3,6-10, 13-14 |
| Y | paragraphs [0023] to [0119]; fig. 1 to 17 (Family: none) | 2,4-5,11-12 |
| Y | WO 2011/037991 A1 (MOUNT SINAI SCHOOL OF MEDICINE), 31 March 2011 (31.03.2011), page 11, lines 11 to 13 & US 2011/0071443 A1 & EP 2480290 A0 | 2,4-5,11-12 |

☒ Further documents are listed in the continuation of Box C.　　☐ See patent family annex.

| | |
|---|---|
| *　Special categories of cited documents: | "T"　later document published after the international filing date or priority date and not in conflict with the application but cited to understand the principle or theory underlying the invention |
| "A"　document defining the general state of the art which is not considered　to be of particular relevance | |
| "E"　earlier application or patent but published on or after the international filing date | "X"　document of particular relevance; the claimed invention cannot be considered novel or cannot be considered to involve an inventive step when the document is taken alone |
| "L"　document which may throw doubts on priority claim(s) or which is cited to establish the publication date of another citation or other special reason (as specified) | "Y"　document of particular relevance; the claimed invention cannot be considered to involve an inventive step when the document is combined with one or more other such documents, such combination being obvious to a person skilled in the art |
| "O"　document referring to an oral disclosure, use, exhibition or other means | |
| "P"　document published prior to the international filing date but later than the priority date claimed | "&"　document member of the same patent family |

| Date of the actual completion of the international search | Date of mailing of the international search report |
|---|---|
| 13 July 2017 (13.07.17) | 25 July 2017 (25.07.17) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2017/018216

C (Continuation).   DOCUMENTS CONSIDERED TO BE RELEVANT

| Category* | Citation of document, with indication, where appropriate, of the relevant passages | Relevant to claim No. |
|---|---|---|
| Y | Takashi Ono, A Study on Brain-Machine Interface Rehabilitation for Stroke Hemiplegia, Graduate School of Science and Technology Keio University, [online], 2015.03, [retrieval date 2017.7.13], Internet: <URL:http://koara.lib.keio.ac.jp/xoonips/modules/xoonips/detail.php?koara_id=KO50002002-20144235-0003>,Chapter 2, Chapter 5, Figure 2-2 | 11-12 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014104549 A